# EUROPEAN PATENT APPLICATION

(11) **EP 2 500 909 A1**
(43) Date of publication of application: **19.09.2012**
(21) Application number: 11158475.1
(22) Date of filing: 16.03.2011
(51) Int. Cl.: G21K 1/04

(54) **Position sensoring method and system for a multi-leaf collimator**

(71) Applicant: Deutsches Krebsforschungszentrum Stiftung des Öffentlichen Rechts, 69120 Heidelberg (DE)
(72) Inventor: Seeber, Steffen, 69121, Heidelberg (DE); Schewiola, Klaus, 69126, Heidelberg (DE)
(74) Representative: Lucke, Andreas

(57) **Abstract**

A multi-leaf collimator for radiation therapy in which the position of the leaves (10;10-1...10-20) are determined by measuring a magnetic field allows to determine the leaf position with enhanced precision, and is at the same time robust to perturbations or disturbances. The magnetic sensor (12,14) may comprise a magnetic encoder (14) that varies in a predefined pattern along a lengthwise direction of the magnetic element (12), in particular according to a step function.

## Description

### FIELD OF THE INVENTION

The present invention relates to a method and system for measuring the position of a leaf in a multi-leaf collimator for radiation therapy.

### BACKGROUND AND RELATED STATE OF THE ART

A multi-leaf collimator is used on a linear accelerator to shape radiotherapy treatment beams. In radiation therapy, a tumour may be irradiated with high energy beams, such as gamma, X-ray, electron or photon radiation from a linear accelerator. The multi-leaf collimator includes a plurality of radiation blocking elements (leaves), usually made of tungsten or some other high atomic numbered material, that can be displaced relative to one another and can be moved independently in and out of the beam path of the treatment beam. When suitably positioned, the leaves together define an aperture with a contour corresponding to the contour of the tumour to be treated. As a result, only the tumour is irradiated with the high-energy treatment beam, not the adjacent healthy body tissue.

Two sets of leaves may be arranged to face each other, such that their end faces can be moved towards and away from each other. The leaves may also be grouped into more than two sets. As a result, virtually any tumour contour may be modelled for conformal radiotherapy.

For intensity-modulated radiation therapy (IMRT), the leaves of a multi-leaf collimator can be moved across the field to create the desired flux or intensity modulation while the treatment beam is switched on or during beam off time (step and shoot mode).

Each of the leaves may be individually displaced by means of an actuator unit (e.g., an electric motor). During the positioning of a leaf, slight deviations of the actual leaf position from the desired or preset value may occur. If this happens, the aperture defined by the leaves may no longer precisely match the borders of the target tumour, so that, by mistake, healthy tissue may be irradiated whereas some parts of the tumour are not irradiated at all.

In order to detect and to correct deviations of the leaf position from the set value, conventional multi-leaf collimators have for some time been equipped with position measurement devices that allow to determine the actual leaf position, and hence to correct for such deviations. This may be achieved by means of a control loop that determines the current leaf position and compensates for a loss of position, for instance a loss of position due to gravitation.

US patent 5,889,834 describes a multi-leaf collimator wherein the individual leaves are provided with oblong connecting cords facing away from the beam side and each engaging a respective position measurement apparatus. The position measurement apparatuses require a lot of space, and hence the connecting cords spread out in a fan shape, similar to a mechanical typewriter. Such a multi-leaf collimator is rather bulky. In addition, the mechanical connections of the individual position measurement apparatuses to the leaves are intricated and prone to measurement inaccuracies. As a result of the indirect transmission mechanism, gear transmission errors may occur and may degrade the measurement precision.

United States patent application US 2010/0008472 A1 describes a measurement apparatus employing a potentiometer arranged at the plate holders of the individual leaves, and hence closer to the leaves than in US 5,889,834. However, the potentiometers take up considerable space. The current leaf position is determined by means of a pick-up of a measuring head dragged along a contact element mounted to the leaf. Since it relies on mechanical contact, this measurement system is susceptible to wear, which may result in measurement inaccuracies. In addition, potentiometers require a complex signal compensation and calibration in order to yield accurate measurement results, again adding to the overall complexity and size of the collimator system.

Optical detectors have likewise been proposed for detecting the leaf position, but are very sensitive to perturbations and radiation, and hence require special casing or shielding, which again adds to the size and complexity of the collimator system.

Modern collimator systems have a large number of fine leaves that allow to model the contour of the target tumour with increasing precision. Hence, the accurate sensing of the leaves positions correspondingly becomes more and more important. Dynamic IMRT, gating and tracking techniques as well as DAO treatments require particularly high precision.

It is thus the objective of the present invention to provide an improved system and method for determining the leaf position, which is both robust and small, but allows to determine the leaf position with high precision.

### OVERVIEW OF THE PRESENT INVENTION

The present invention achieves this objective with a position sensoring system and method with the features of independent claims 1 and 14, respectively. The dependent claims relate to preferred embodiments.

A multi-leaf collimator according to the present invention comprises a plurality of leaves that are adapted to be moved in a movement direction in and out of a beam path and to define a preset contour, as well as a measuring device for determining a position of at least one leaf along said movement direction, wherein said measurement device is adapted to determine said position by measuring a magnetic field.

A measurement device adapted to determine the leaf position by means of measuring a magnetic field has the advantage of being rather insensitive to radiation. This allows to position the measurement device close to or even at the leaf, thereby avoiding gear transmission errors or inaccuracies that may accumulate when the leaf movement has to be converted for measurement at a remote site, as in the prior art.

According to a preferred embodiment, said measurement device may be a magnetic sensor, in particular a Hall effect sensor.

Magnetic sensors also allow for contact-free measurements, and therefore measurement results are not affected by friction or abrasive wear.

A magnetic sensor can be made very small, and hence each individual leaf may be equipped with its own sensor device, even in modem multi-leaf collimator systems with a large number of small leaves. At the same time, a magnetic sensor allows to determine the position of the leaves with high accuracy.

Since the measurement device relying on the measurement of a magnetic field is small, it can be integrated into a leaf with minimal impact on the penumbra, i.e. on the shielding properties of the leaves. As a consequence, a measurement device according to the present invention can be employed in virtually any modem multi-leaf collimator, and requires neither an extensive redesign of the leaf system nor amendments to its mode of operation during therapy. Hence, the system and method according to the present invention are well-suited to upgrade existing collimator systems.

In particular, said leaf may comprise at least part of said measurement device. Preferably, said part of said measurement device may be integrated into said leaf, in particular milled into said leaf. This allows to provide said leaf with the measurement device with minimal impact on the operability of the collimator.

Alternatively, said part of said measurement device may be attached to said leaf, in particular mounted onto a surface of said leaf or glued to said leaf. Since the magnetic sensor is small, it may be attached to the outer surface of the leaf without affecting the operability of the collimator system. In particular, this may allow existing collimator systems to be upgraded easily with a measurement device according to the present invention, and at low costs.

In a preferred embodiment, said measurement device comprises a magnetic element adapted to provide a magnetic field, and a detector device capable of detecting said magnetic field, wherein said leaf comprises one of said magnetic element and said detector device, and the other of said magnetic element and said detector device is attached to a supporting device relative to which said leaf is movable along said movement direction.

The magnetic field provided by the magnetic element may vary along said movement direction, and hence the detector device will detect a magnetic field that may be indicative of the current leaf position when the position of the leaf (and thus the relative position between magnetic element and detector element) is changed during movement of the leaf along the movement direction.

Said supporting device may form part of the system for driving said leaf, or may form a separate means for mounting at least part of said measurement device. It may be any device relative to which said leaf moves along said movement direction.

The measurement device of the multi-leaf collimator according to the present invention may comprise a magnetic element adapted to provide a magnetic field, and a detector device capable of detecting said magnetic field, wherein said leaf comprises one of said magnetic element and said detector device at an upper face of said leaf, and the other of said magnetic element and said detector device is attached to a supporting device facing said upper face, wherein said leaf is movable relative to said supporting device in said movement direction.

An upper face in the sense of the latter embodiment may be a face of the leaf that points towards the radiation source.

The measurement device may also comprise a magnetic element adapted to provide a magnetic field, and a detector device capable of detecting said magnetic field, wherein said leaf comprises one of said magnetic element and said detector device at a lower face of said leaf, and the other of said magnetic element and said detector device is attached to a supporting device facing said lower face, wherein said leaf is movable relative to said supporting device in said movement direction.

A lower face in the sense of the latter embodiment may be a face of the leaf that points away from the radiation source, and may be opposite to said upper face.

A supporting device facing an upper or lower end of said leaf may conveniently be integrated into a driving system for said leaf.

Alternatively or additionally, said measurement device may also comprise a magnetic element adapted to provide a magnetic field, and a detector device capable of detecting said magnetic field, wherein said leaf comprises one of said magnetic element and said detector device at a side face of said leaf, and the other of said magnetic element and said detector device is attached to a supporting device facing said side face, wherein said leaf is movable relative to said supporting device in said movement direction.

A side face in the sense of the latter embodiment may be a face of the leaf that points in a direction perpendicular to the propagation of the radiation beam.

A multi-leaf collimator according to the latter embodiment has the additional advantage of minimizing the interference of the measurement device with the driving means for the leaf, which may typically engage with the leaf at their upper and/or lower face.

Preferably, said magnetic element or said detector device may be integrated into said leaf, in particular milled into said leaf.

Alternatively, said magnetic element or said detector device may be attached to said leaf, in particular glued to said leaf.

In a preferred embodiment, said magnetic element is a magnetic encoder adapted to provide a static magnetic field that varies in a predefined pattern along a lengthwise direction of said magnetic element. In particular, said predefined pattern may be a step function.

A predefined pattern according to the latter embodiment may be a characteristic pattern that allows to determine the current leaf position even in the presence of magnetic stray fields, or a large magnetic background. In particular, a predefined pattern that varies as a step function along the lengthwise direction of the magnetic element allows to directly extract from said magnetic sensor a digital signal indicative of the current detector position, without any complex and expensive analog-to-digital conversion. Likewise, a measurement device according to the latter embodiment does not need complex signal compensation or calibration, as is the case for conventional measurement devices such as potentiometers.

A plurality of leaves of the multi-leaf collimator may each be equipped with a measurement device according to any one of the previously described embodiments of the present invention. This allows to detect each leaf position individually, thereby greatly enhancing the accuracy for modelling the contour of the target tumour.

A multi-leaf collimator according to a preferred embodiment has a plurality of measurement devices for measuring the position of a plurality of leaves along said movement direction, wherein said measurement device comprises a magnetic element adapted to provide a magnetic field and a detector device capable of detecting said magnetic field. Each said leaf may comprise one of said magnetic elements and said detector devices, and the other of said magnetic elements and said detector devices may be attached to a common supporting device relative to which said leaves are movable along said movement direction.

By supporting said magnetic elements and/or detector devices from a common supporting device, the position of a plurality of leaves can be detected reliably with a detection system that requires little space and does not interfere with the driving means for driving said leaves.

In particular, said common supporting device may comprise a plurality of extensions mounted to a common pole bridging said leaf wherein each said extension extends into the space between two neighbouring leaves and carries at least one of said magnetic elements or detector devices.

The present invention also relates to a method for measuring the position of at least one leaf in a multi-leaf collimator for radiation therapy appliances, comprising the step of determining the position of said leaf along a movement direction by measuring a magnetic field.

In a preferred embodiment, said method further comprises the step of providing a static magnetic field that varies along said movement direction.

Said position may be determined by means of the measurement device with some or all of the features described above with reference to the multi-leaf collimator system according to the present invention.

### DETAILED DESCRIPTION OF PREFERRED EMBODIMENTS

The characteristics and numerous advantages of the present invention will become best apparent from a detailed description of the accompanying drawings, in which:
- Fig. 1a: is a schematic side view of a leaf with a measurement device according to a first embodiment of the present invention;
- Fig. 1b is: a schematic view of the leaf and measurement device shown in Fig. 1a;
- Fig. 2: is a cross-sectional view of a leaf and measurement device according to a second embodiment of the present invention;
- Fig. 3: is a schematic drawing illustrating a Hall sensor with magnetic encoder that may be used in a measurement device according to the present invention;
- Fig. 4a: is a schematic side view of a leaf and measurement device according to a third embodiment of the present invention;
- Fig. 4b: is a schematic cross-sectional view of the leaf and measurement device shown in Fig. 4a;
- Fig. 5: is a schematic cross-sectional view of a leaf and measurement device according to a fourth embodiment of the present invention;
- Fig. 6a: is a schematic side view of a leaf and measurement device according to a fifth embodiment of the present invention;
- Fig. 6b: is a schematic cross-sectional view of the leaf and measurement device shown in Fig. 6a;
- Fig. 7: is a schematic cross-sectional view of a leaf and measurement device according to a sixth embodiment of the present invention;
- Fig. 8a: is a schematic side view of a leaf and measurement device according to a seventh embodiment of the present invention;
- Fig. 8b: is a schematic cross-sectional view of a leaf and measurement device according to the embodiment shown in Fig. 8a;
- Fig. 9: is a schematic cross-sectional view of a leaf and measurement device according to an eighth embodiment of the present invention;
- Fig. 10a: is a schematic cross-sectional view of a leaf and measurement device according to a ninth embodiment of the present invention;
- Fig. 10b: is a schematic cross-sectional view of a leaf and measurement device according to a tenth embodiment of the present invention;
- Fig. 11a: is a schematic cross-sectional view of a leaf and measurement device according to a eleventh embodiment of the present invention, which is an alternative to the embodiment shown in Fig. 10a;
- Fig. 11b: is a schematic cross-sectional view of a leaf and measurement device according to a twelfth embodiment of the present invention, which is an alternative to the embodiment shown in Fig. 10b;
- Fig. 12a: is a schematic cross-sectional view of a leaf and measurement device according to a thirteenth embodiment of the present invention;
- Fig. 12b: is a schematic cross-sectional view of a leaf and measurement device according to a fourteenth embodiment of the present invention;
- Fig. 13a: is a schematic cross-sectional view of a leaf and measurement device according to a fifteenth embodiment of the present invention;
- Fig. 13b: is a schematic cross-sectional view of a leaf and measurement device according to a sixteenth embodiment of the present invention;
- Fig. 14a: is a perspective drawing illustrating a plurality of leaves and corresponding measurement devices arranged along a common support structure; and
- Fig. 14b: is an enlarged blow-up of part of the configuration shown in Fig. 14a.

The present invention may be employed with any multi-leaf collimator for conformal or intensity modulated radiation therapy. Such collimators are well-known in the art and comprise a plurality of leaves that can be moved in a movement direction in and out of treatment beam paths so as to collectively define a preset contour that matches the contour of the target tumour. Examplary reference is made to US 5,889,834 as well as US 2010/0008472 A1, which describe the design and functionality of such conventional multi-leaf collimators in further detail.

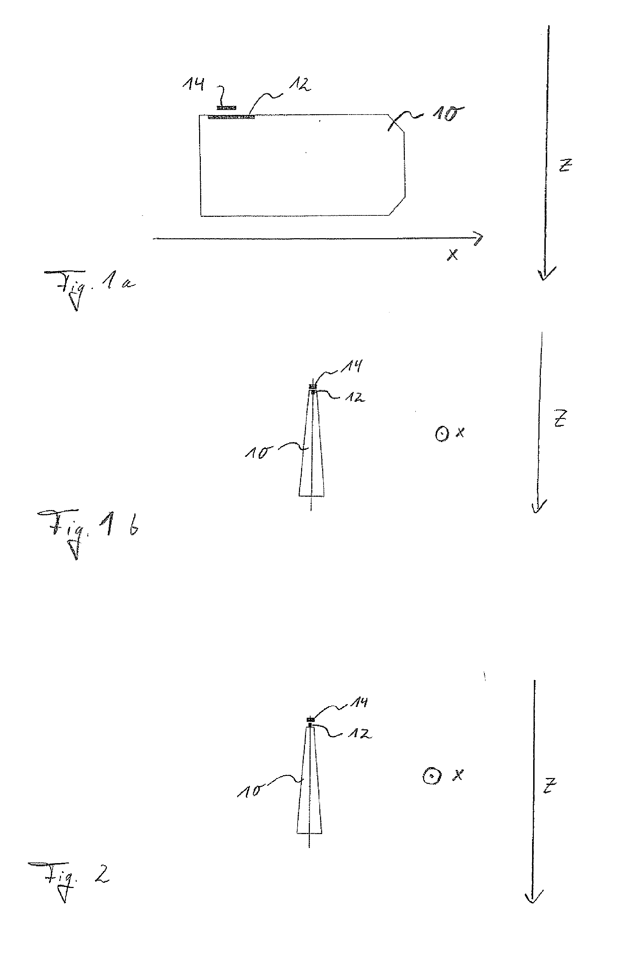

Fig. 1a shows a schematic side view of a leaf 10 that may be one of a plurality of leaves in a multi-leaf collimator according to the present invention. Leaf 10 may be moved in a movement direction indicated by arrow x by means of a motor or other driving means (not shown). By moving leaf 10 in direction x, the leaf 10 may be placed in the beam path to block a portion of said beam. The incident direction of the beam is indicated by arrow z in Fig. 1a.

A possibly large number of further leaves of identical or similar configuration may be arranged in parallel to leaf 10 to form a first set. A second set of leaves may be arranged in an opposed relationship to said first set and may likewise be movable back and forth along direction x. By suitably moving the leaves in direction x, the collection of leaves cut a preset contour out of the beam travelling in direction z, and said contour may be chosen to correspond to the contour of the target tumour.

Fig. 1a further depicts a measurement device comprising a magnetic element 12 on the upper face (pointing towards the radiation source) of the leaf 10 as well as a detector device 14 held by a supporting structure (not shown) in the vicinity above the upper face of leaf 10. Fig. 1b shows a cross-sectional view of the same leaf 10 and measurement device comprising magnetic element 12 and detector device 14, viewed along direction x. The leaf shown in Figs. 1a and 1b (as well as in the further embodiments described below) has a cross-section that forms a truncated cone. However, it is an advantage of the present invention that it may be practiced with leaves of virtually any shape.

The magnetic element 12 may be a magnetic strip with a magnetic field that varies in a predefined pattern along the lengthwise direction (corresponding to direction x) of the magnetic element 12.

The detector device 14 may be a detector capable of detecting the magnetic field generated by magnetic element 12, and in particular may sense the variations of the magnetic field when the magnetic element 12 and the detector device 14 are moved relative to one another along direction x. In particular, detector device 14 may be a Hall detector.

Hence, as the leaf 10 is driven by the driving means (not shown) to move along direction *x* in and out of the beam path, the magnetic element 12 will change its position with respect to the detector device 14 correspondingly, and the detector device 14 will sense a corresponding change in the magnetic field. This allows the detector device 14 to determine the position of leaf 10 along direction x with high precision.

Magnetic sensors allow to measure the position of the leaf 10 with high accuracy. For instance, state-of-the-art Hall sensors may achieve a measurement accuracy in the nanometer range. In addition, the measurement of the magnetic field is contact-free, and hence measurement results are not distorted by mechanical abrasions, such as might be the case in conventional measurement techniques employing potentiometers.

Magnetic sensors are very small and lightweight. For instance, the weight of a measurement device according to the present invention may be below 5 g.

The magnetic element 12 may thus be placed directly at the leaf, even for very small leaves, and hence the position of the leaf may be inferred directly at the leaf. This has the advantage that no gear transmission to a remote measurement device is needed, which reduces the size of the multi-leaf collimator and also eliminates the likelihood of measurement errors due to mechanical play or distortions in the gear transmission, and hence leads to higher positioning precision.

Magnetic sensors are resilient to radiation, and hence may be placed directly at the leaf 10 in the vicinity of the treatment beam path. They are also insensible to dirt, and hence reliable measurements may be obtained for the entire life span of the multi-leaf collimator.

As shown in Figs. 1a and 1b, because of its small size the magnetic strip 12 may be integrated into leaf 10, in particular milled into leaf 10. Hence, the extra space needed to accommodate the measurement device is reduced, which is particularly advantageous in multi-leaf collimators with a large number of small leaves. In more generality, a measurement device that is integrated into a leaf may be a measurement device whose upper surface is flush with the leaf surface.

However, as shown in the schematic front view of Fig. 2, the magnetic element 12 may likewise be attached to the upper face of said leaf 10, and in particular may be glued to the leaf 10. This alternative solution may be equally effective for accurately determining the current position of the leaf 10 along the movement direction x, but has the additional advantage that it allows existing collimator systems to be easily upgraded with a measurement device according to the present invention.

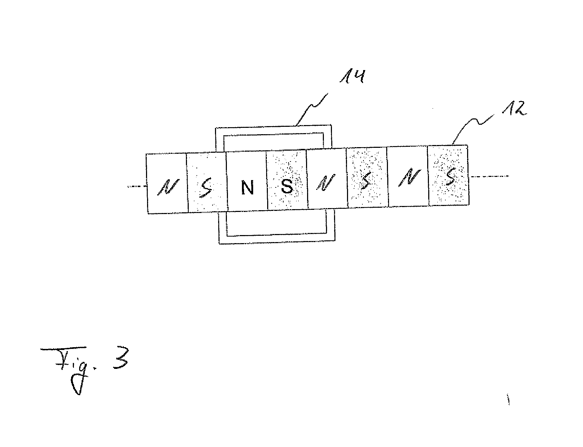

The magnetic element 12 may be a magnetic encoder in which regions of positive and negative magnetization (north poles N and south poles *S*) alternate along a lengthwise direction of said magnetic element (corresponding to the movement direction x), as illustrated in Fig. 3. The resulting magnetization pattern resembles a step function. The magnetic field detected by the detector device (integrated Hall array) 14 when the magnetic element 12 moves relative to the detector device 14 along the movement direction x is hence a differential (quasi-digital) signal, and the number of changes between north and south poles gives an indication of a current leaf position. Since the pattern that can be extracted from a magnetic encoder with a step function is quasi-digital from the start, no subsequent expensive analog-to-digital conversion is necessary. This further reduces the complexity and size of the measurement device according to the present invention. In particular, all the circuitry for analysis of the detector device may be integrated into the detector device 14, and there is no need for extra digitalizing units or complex signal compensation and calibration circuits, as would be the case for standard potentiometers.

However, the measurement resolution is in general not limited to the spatial distance over which north poles N and south poles S vary. In particular, the leaf position may also be determined with high accuracy by means of a magnetic encoder having only relatively few variations of north poles N and south poles S by measuring the magnetic field emanating from these variations, and converting them to a digital signal by means of post-processing in the sensor device.

In any case, the detector device may detect and evaluate the changes of the magnetic field as the magnetic element and the sensor are in relative movement. The encoder may provide the resulting position information already as an industrial standard encoder signal.

Figs. 1a, 1b, and 2 illustrate embodiments in which the magnetic element 12 is mounted onto the leaf 10, while the detector device 14 is stationary. However, it will be apparent to those skilled in the art that similar results may be obtained when the detector device 14 is mounted to the leaf, whereas the magnetic element 12 is supported by the stationary supporting device (not shown).

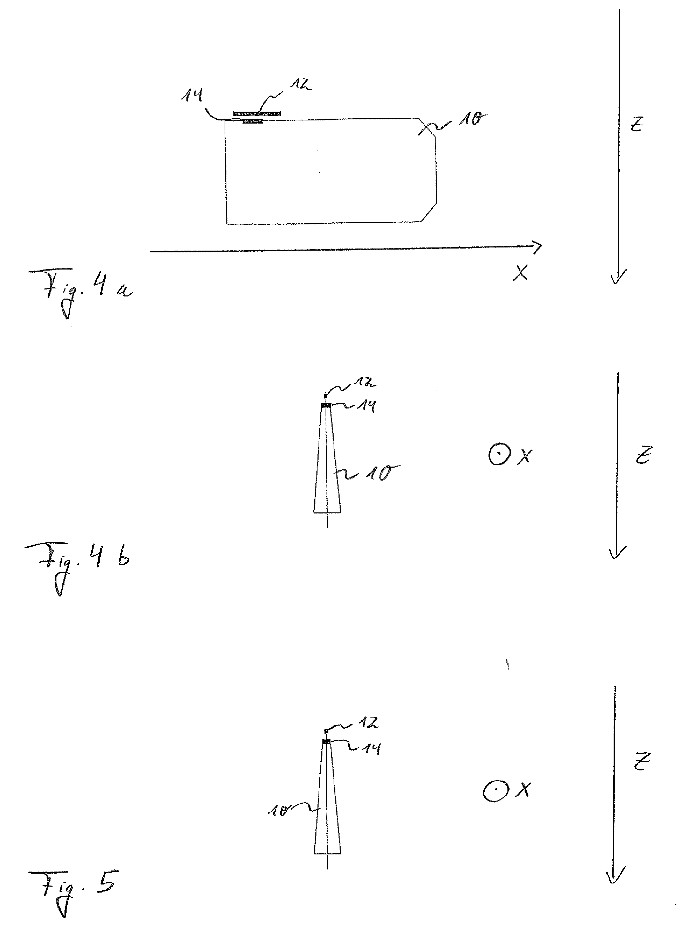

Such alternative embodiments are shown in Figs. 4a, 4b and 5. In the embodiment according to Figs. 4a and 4b, the detector device 14 is integrated into the upper face of leaf 10. Fig. 5 shows a very similar configuration, but with a detector device 14 glued to the upper surface of leaf 10.

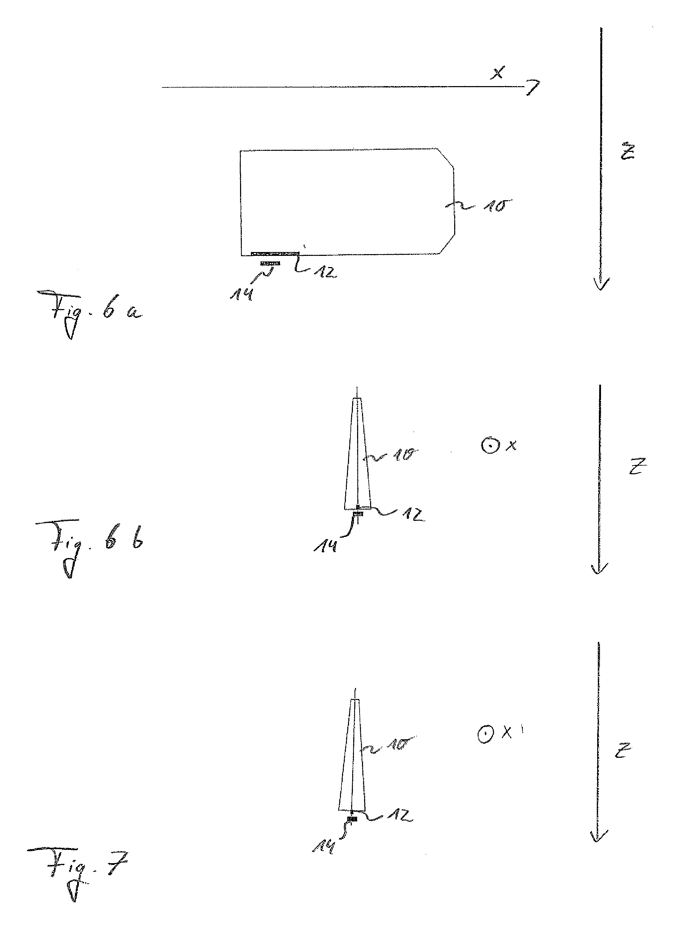

In the previous embodiments, the measurement device is positioned at an upper face of the leaf 10, i.e. a face of the leaf 10 facing the radiation source. However, as depicted in Figs. 6a to 9, the measurement device may likewise be positioned at a lower face of the leaf 10. In the embodiment shown in Figs. 6a and 6b, the magnetic element 12 is milled into the lower face of leaf 10, whereas the detector device 14 is stationary and is attached to a supporting device (not shown) facing said lower face.

The embodiment shown in Fig. 7 is very similar, with the only exception that the magnetic element 12 is glued to a lower surface side of leaf 10, said lower surface side facing the detector device 14.

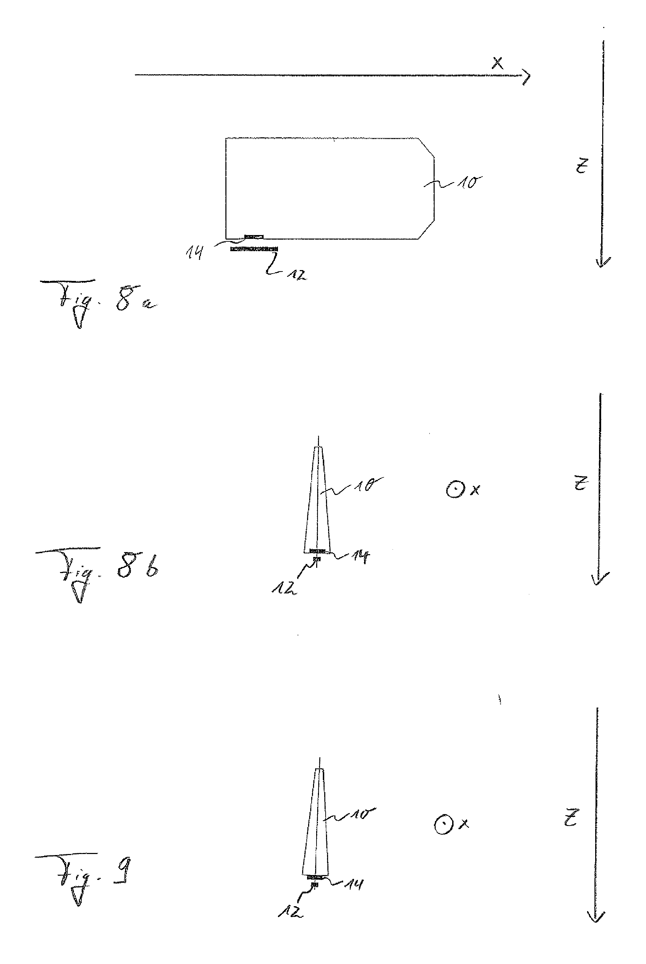

The embodiments shown in Figs. 8a, 8b, and 9 correspond to the embodiments described previously with reference to Figs. 6a, 6b, and 7, respectively, with the only exception that the positions of magnetic element 12 and detector device 14 are interchanged. Hence, in the embodiment described with reference to Fig. 8a and 8b, the detector device 14 is milled into the surface of the lower face of leaf 10, while the magnetic element 12 is stationary and is attached to a supporting device (not shown) opposite said lower face of leaf 10. The embodiment described with reference to Fig. 9 is very similar, with the only exception that the detector device 14 is mounted onto the surface of the lower face of leaf 10, for instance glued onto said surface.

The upper and lower face of leaf 10 may likewise be employed to engage the driving means (not shown) for driving the leaf 10 in the movement direction x. In this case, it may be possible to integrate the supporting device into said driving means, thereby further reducing the size of the multi-leaf collimator.

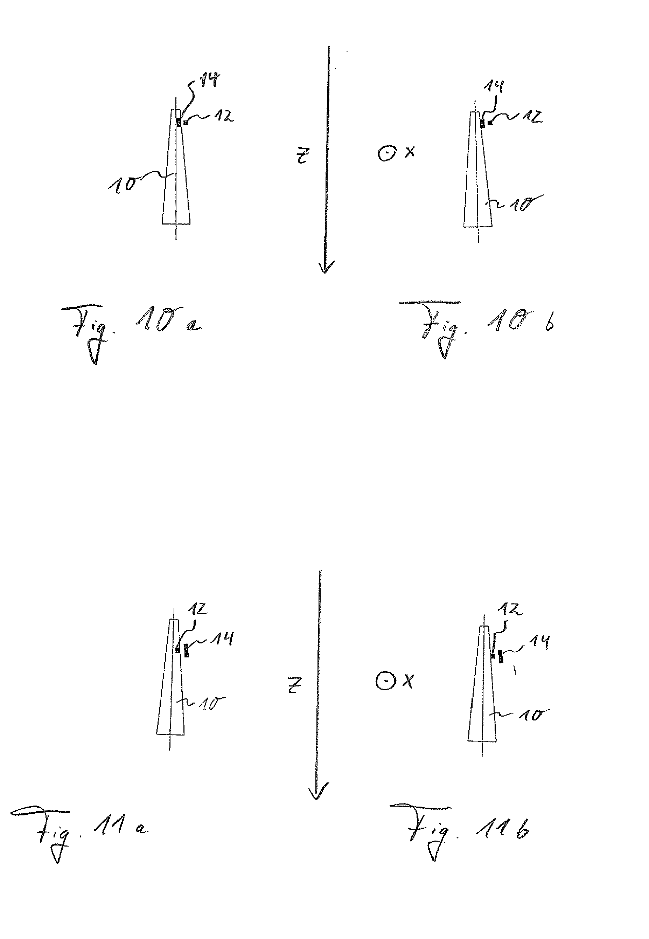

However, the measurement device may likewise be positioned at a side face of leaf 10, as shown in Figs. 10a to 13b.

Fig. 10a depicts an embodiment in which the detector device 14 is milled into a right-hand side face of leaf 10 (when viewed against direction x), whereas the magnetic element 12 is stationary and is attached to said supporting device (not shown) in the vicinity of said side face.

Fig. 10b shows a corresponding configuration, with the only exception that the detector device 14 is not integrated into the side face of leaf 10, but is glued onto the surface of the side face of leaf 10.

Figs. 11a and 11b largely correspond to the embodiments described above with reference to Figs. 10a and 10b, respectively, with the only exception that the positions of magnetic element 12 and detector device 14 are interchanged. Hence, with reference to Fig. 11a, the magnetic element 12 may be integrated into the side face of leaf 10, while the detector device 14 is stationary and is attached to a supporting device (not shown) facing said side face of leaf 10. Fig. 11b shows a corresponding embodiment, with the only exception that magnetic element 12 is mounted onto the upper surface of the side face of leaf 10, for instance glued onto said surface.

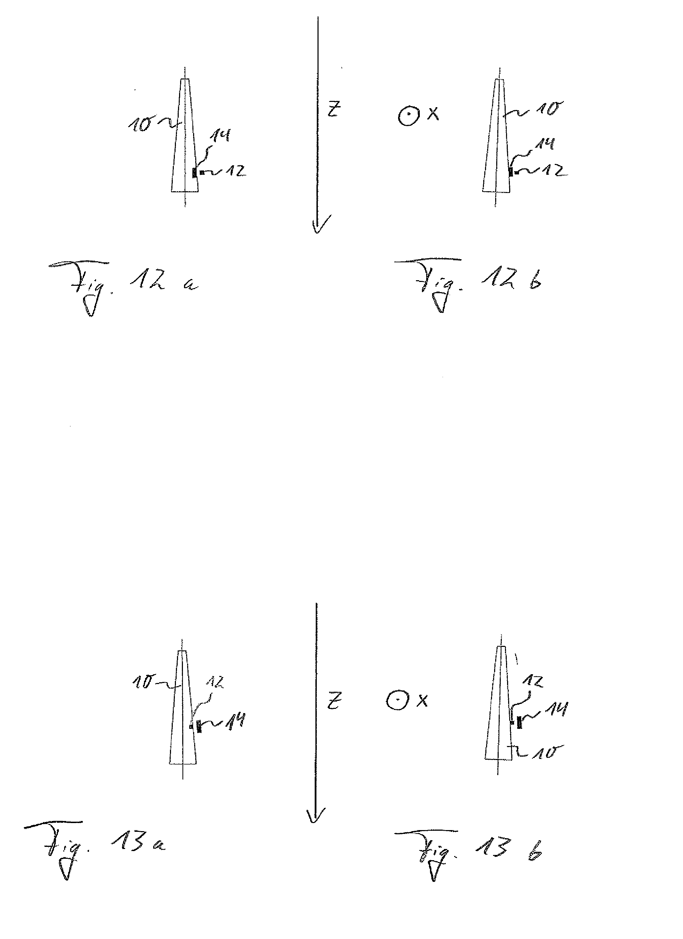

In the embodiments described above with reference to Figs. 10a to 11b, the measurement device is positioned towards the upper end of the side face of leaf 10, i.e. towards the radiation source. However, it will be apparent to those skilled in the art that the measurement device may be positioned anywhere along the side face of leaf 10. In particular, Figs. 12a to 13b show corresponding embodiments in which a measurement device comprising magnetic element 12 and detector device 14 is positioned towards the lower end of leaf 10, away from the radiation source.

Since a magnetic sensor is small and lightweight, the present invention allows each of the leaves of a multi-leaf collimator to be equipped with its own measurement device, so that the position of each leaf can be determined individually, even if the leaves are small. This results in a multi-leaf collimator that allows to be set to varying tumour contours with enhanced precision.

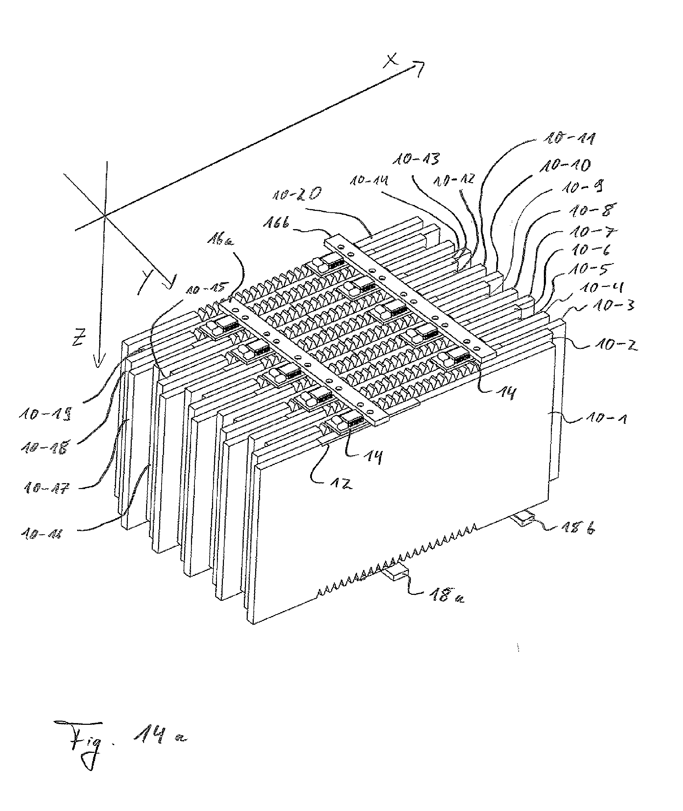

A configuration employing a plurality of leaves with corresponding measurement devices is shown schematically in Fig. 14a. Fig. 14a shows a stack of twenty leaves 10-1 to 10-20 arranged in parallel, but in principle any number of leaves may be combined into a multi-leaf collimator according to the present invention. A first upper bridge 16a and a second upper bridge 16b span the stack of leaves 10-1 to 10-20 at an upper side thereof, facing said radiation source. Each of said upper bridges 16a, 16b carries a plurality of detector devices 14 as described above with reference to Figs. 1a and 1b. The leaves have magnetic strips 12 serving as magnetic elements mounted to an upper face, facing said detector devices 14. As can be taken from Fig. 14a, upper bridge 16a carries detector devices 14 for leaves 10-1, 10-5, 10-9, 10-13, and 10-17, whereas upper bridge 16b carries detector devices 14 for leaves 10-3, 10-7, 10-11, 10-15, and 10-19.

Detector devices for leaves 10-2, 10-6, 10-10, 10-14, and 10-18 are carried by a lower bridge 18a spanning the stack of leaves 10-1 to 10-20 at a lower side thereof. The remaining detector devices for leaves 10-4, 10-8, 10-12, 10-16, and 10-20 are carried by second lower bridge 18b likewise spanning the stack of leaves 10-1 to 10-20 at a lower side thereof.

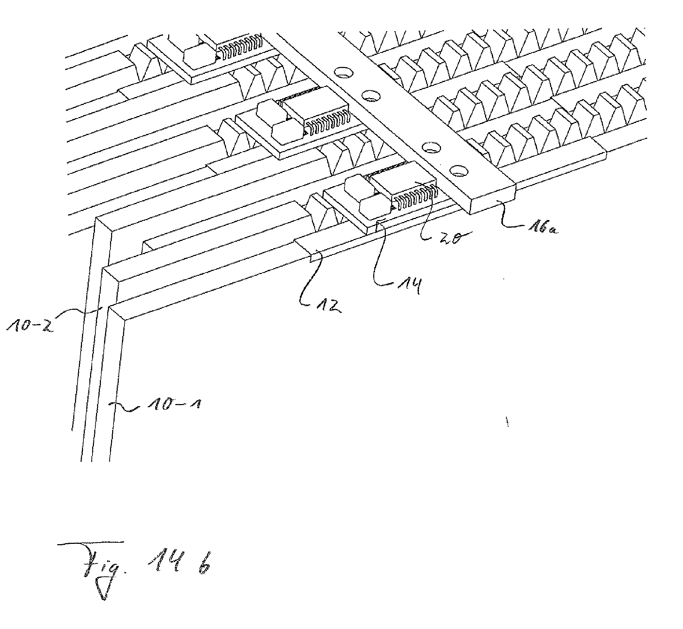

An enlarged cut-out showing the upper bridge 16a and detector devices 14 and magnetic strips 12 in additional detail is depicted in Fig. 14b. A Hall sensor chip 20 employed for reading out the magnetic strip 12 comprises all the digitalization circuitry, and hence directly yields a digital signal indicative of the position of leaf 10-1 along the movement direction x.

In Figs. 14a and 14b, the Hall sensor chip 20 is placed on an upper side of the detector device 14. However, the Hall sensor chip 20 may likewise be placed at a lower side thereof, facing the magnetic strip 12.

The configuration in which the detector devices 14 are distributed over upper bridges 16a, 16b and lower bridges 18a, 18b allows to provide each leaf 10-1, ..., 10-20 with its individual measurement device while still maintaining a minimal distance between adjacent leaves. At the same time, the configuration shown in Fig. 14a ensures a maximal distance between any two measurement devices, thereby avoiding crosstalk that may compromise the measurement accuracy.

The drawings and preferred embodiments merely serve to explain and illustrate the invention and the numerous advantages it entails, but should not be understood to limit the invention in any sense. The scope of the invention is to be determined solely by the appended set of claims.

### List of reference signs

- 10: leaf
- 10-1, ..., 10-20: leaves
- 12: magnetic element
- 14: detector device
- 16a, 16b: upper bridges
- 18a, 18b: lower bridges
- 20: Hall sensor chip

## Claims

1. A multi-leaf collimator for radiation therapy appliances with:
a plurality of leaves (10; 10-1,...,10-20) that are adapted to be moved in a movement direction (x) in and out of a beam path and to collectively define a preset contour; and
a measurement device (12, 14) for determining a position of at least one leaf (10; 10-1,...,10-20) along said movement direction (x);
wherein said measurement device (12, 14) is adapted to determine said position by measuring a magnetic field.

2. The multi-leaf collimator according to claim 1, wherein said measurement device is a magnetic sensor (12, 14), in particular a Hall effect sensor.

3. The multi-leaf collimator according to claim 1 or 2, wherein said leaf (10; 10-1, ..., 10-20) comprises at least part of said measurement device (12, 14).

4. The multi-leaf collimator according to any of the preceding claims, wherein said measurement device comprises a magnetic element (12) adapted to provide a magnetic field, and a detector device (14) capable of detecting said magnetic field, wherein said leaf (10; 10-1,...,10-20) comprises one of said magnetic element (12) and said detector device (14), and the other of said magnetic element (12) and said detector device (14) is attached to a supporting device relative to which said leaf (10; 10-1,...,10-20) is movable along said movement direction (x).

5. The multi-leaf collimator according to any of the preceding claims, wherein said measurement device comprises a magnetic element (12) adapted to provide a magnetic field, and a detector device (14) capable of detecting said magnetic field, wherein said leaf (10; 10-1,...,10-20) comprises one of said magnetic element (12) and said detector device (14) at an upper face of said leaf (10; 10-1,...,10-20), and the other of said magnetic element (12) and said detector device (14) is attached to a supporting device facing said upper face, wherein said leaf (10; 10-1,...,10-20) is movable relative to said supporting device in said movement direction (x).

6. The multi-leaf collimator according to any of the preceding claims, wherein said measurement device comprises a magnetic element (12) adapted to provide a magnetic field, and a detector device (14) capable of detecting said magnetic field, wherein said leaf (10; 10-1,...,10-20) comprises one of said magnetic element (12) and said detector device (14) at a lower face of said leaf (10; 10-1,...,10-20), and the other of said magnetic element (12) and said detector device (14) is attached to a supporting device facing said lower face, wherein said leaf (10; 10-1,...,10-20) is movable relative to said supporting device in said movement direction (x).

7. The multi-leaf collimator according to any of the preceding claims, wherein said measurement device comprises a magnetic element (12) adapted to provide a magnetic field, and a detector device (14) capable of detecting said magnetic field, wherein said leaf (10; 10-1,...,10-20) comprises one of said magnetic element (12) and said detector device (14) at a side face of said leaf (10; 10-1,...,10-20), and the other of said magnetic element (12) and said detector device (14) is attached to a supporting device facing said side face, wherein said leaf (10; 10-1,...,10-20) is movable relative to said supporting device in said movement direction (x).

8. The multi-leaf collimator according to any of the claims 4 to 7, wherein said magnetic element (12) or said detector device (14) are integrated into said leaf (10; 10-1,...,10-20), in particular milled into said leaf (10; 10-1,...,10-20).

9. The multi-leaf collimator according to any of the claims 4 to 7, wherein said magnetic element (12) or said detector device (14) are attached to said leaf (10; 10-1,...,10-20), in particular mounted to an outer surface of said leaf (10; 10-1, ...,10-20) or glued to said leaf (10; 10-1,...,10-20).

10. The multi-leaf collimator according to any of the claims 4 to 9, wherein said magnetic element (12) is a magnetic encoder adapted to provide a static magnetic field which varies in a predefined pattern along a lengthwise direction of said magnetic element (12).

11. The multi-leaf collimator according to claim 10, wherein said predefined pattern is a step function.

12. The multi-leaf collimator according to any of the preceding claims with a plurality of measurement devices for measuring the position of a plurality of leaves (10; 10-1,...,10-20) along said movement direction (x), wherein each said measurement device comprises a magnetic element (12) adapted to provide a magnetic field and a detector device (14) capable of detecting said magnetic field, wherein each leaf (10; 10-1,...,10-20) comprises one of said magnetic elements (12) and said detector devices (14), and the other of said magnetic elements (12) and said detector devices (14) is attached to a common supporting device (16a, 16b, 18a, 18b) relative to which said leaves (10; 10-1,...,10-20) are movable along said movement direction (x).

13. The multi-leaf collimator according to claim 12, wherein said common supporting device (16a, 16b, 18a, 18b) comprises a plurality of extensions mounted to a common pole bridging said leaves (10; 10-1,...,10-20), each said extension extending into the space between two neighbouring leaves (10; 10-1,...,10-20) and carrying at least one of said magnetic elements (12) or said detector devices (14).

14. A method for measuring the position of at least one leaf (10; 10-1,...,10-20) in a multi-leaf collimator for radiation therapy appliances, comprising the step of determining the position of said leaf (10; 10-1,...,10-20) along a movement direction (x) by measuring a magnetic field.

15. The method according to claim 14, further comprising the step of providing a static magnetic field that varies along said movement direction (x).
